# EUROPÄISCHE PATENTSCHRIFT

(11) **EP 3 132 252 B1**
(45) Veröffentlichungstag und Bekanntmachung des Hinweises auf die Patenterteilung: **02.04.2025**
(21) Anmeldenummer: 15717167.9
(22) Anmeldetag: 17.04.2015
(51) Int. Cl.: G01N 21/90, A61L 2/10, A61L 2/28, B08B 9/46

(54) **BEHÄLTERINSPEKTION**
INSPECTION OF CONTAINERS
INSPECTION DE CONTENANTS

(30) Priorität: 17.04.2014 DE 102014005650
(43) Veröffentlichungstag der Anmeldung: 22.02.2017
(73) Patentinhaber: Heuft Systemtechnik GmbH, 56659 Burgbrohl (DE)
(72) Erfinder: HEUFT, Bernhard, 56659 Burgbrohl (DE)
(74) Vertreter: Abitz & Partner
(86) Internationale Anmeldenummer: PCT/EP2015/058418
(87) Internationale Veröffentlichungsnummer: WO 2015/158907

(56) Entgegenhaltungen:
- EP-A1- 1 614 630
- WO-A1-88/00862
- WO-A1-89/09391
- DE-A1- 4 223 269
- GB-A- 1 182 413
- JP-A- S52 135 788

## Beschreibung

Die Erfindung betrifft ein Verfahren und eine Vorrichtung zur Inspektion von leeren Behältern auf Verunreinigungen zum Einsatz in automatischen Abfüllanlagen, in denen die Behälter mit hohen Geschwindigkeiten von bis zu 90.000 Flaschen pro Stunde transportiert werden, mit einer Strahlungsquelle zur Generation einer anregenden Strahlung, wobei die anregende Strahlung auf die Innenwand eines leeren Behälters geleitet wird und dort zu detektierende Verunreinigungen zur Abgabe von Lumineszenz-Strahlung anregt, mit mindestens einer Einrichtung zur Detektion der von den Verunreinigungen abgegebenen Lumineszenz-Strahlung, sowie mit einer Einrichtung zur Auswertung der detektierten Lumineszenz-Strahlung.

Die vorliegende Erfindung ist zum Einsatz in automatischen Abfüllanlagen bestimmt, in denen die Behälter mit hohen Geschwindigkeiten von bis zu 90.000 Flaschen pro Stunde transportiert werden. Um den Durchsatz derartiger Flaschenabfüllanlagen nicht zu beeinträchtigen, müssen die Inspektionseinrichtungen zur Behälterkontrolle ebenfalls dazu ausgelegt sein, die Behälter mit hohen Geschwindigkeiten zu inspizieren.

In automatischen Abfüllanlagen, werden leere Behälter vor der Befüllung auf mögliche Verunreinigungen oder Fremdkörper untersucht. Herkömmlicherweise werden die Behälter hierzu durch eine Inspektionseinrichtung geführt, die eine Lichtquelle für sichtbares Licht und eine CCD-Kamera umfasst. Die Behälter werden dabei im Wesentlichen durchleuchtet und aus unterschiedlichen Blickwinkeln inspiziert, so dass eine verlässliche Erkennung von Verunreinigungen gewährleistet ist. Eine solche Inspektionseinrichtung ist zum Beispiel aus der EP 0 415 154 A1 bekannt.

Es hat sich gezeigt, dass bestimmte Verunreinigungen, insbesondere organische Verunreinigungen wie Schimmelpilze, Fette, Kohlenwasserstoffe, Insektenlarven, Keime, oder Kunststoffe mit herkömmlichen Inspektionseinrichtungen mit solchen Inspektionseinrichtungen nur schwer oder gar nicht detektierbar sind.

Es ist ferner bekannt, dass manche, insbesondere organische Verunreinigungen Lumineszenz-Erscheinungen aufweisen, d.h. dass sie durch Energieeinwirkung von außen in angeregte Zustände versetzt werden können und anschließend durch Aussenden von Lumineszenz-Strahlung wieder in den Grundzustand zurückgelangen können. Die verschiedenen Arten der Lumineszenz werden hierbei nach der Dauer des Leuchtens nach dem Ende der Anregung eingeteilt werden. Als Fluoreszenz wird ein sehr kurzes Nachleuchten bezeichnet, welches als unmittelbare Folge und Begleiterscheinung der Anregung auftritt. Der Begriff Phosphoreszenz beschreibt ein längeres Nachleuchten welches länger als 1 ms nach dem Ende der Anregung anhält.

Üblicherweise erfolgt die Anregung hierbei durch Bestrahlen mit UV-Licht. UV-Licht wird herkömmlicherweise nicht zur Behälterinspektion verwendet, da insbesondere Behälterglas eine sehr geringe oder gar keine Transparenz für UV-Licht aufweist.

Aus der WO 2008/092537 A1 ist eine Vorrichtung zur optischen Charakterisierung von Probenmaterial bekannt, wobei unter anderem auch UV-Licht eingesetzt wird. Mindestens ein UV-Detektor ist vorgesehen mit dem die Fluoreszenz bzw. die Lumineszenz der Probe ermittelt werden kann. Da die Beleuchtung der Probe von außen durch die Behälterwand erfolgt, muss diese transparent für die verwendete Strahlung sein.

Aus der DE 10 2010 043 131 B1 ist eine Vorrichtung zur berührungslosen Untersuchung einer Eigenschaft des Inhalts eines Behälters mittels elektro-magnetischer Strahlung bekannt. Der Behälter kann zum Beispiel ein Getreidesilo, ein Gärtank, oder ein ähnliches Behältnis sein und die Vorrichtung dient dazu eine berührungslose Messung zur Überwachung des Prozessfortschritts des in dem Behälter befindlichen Inhalts durchzuführen. Da die Eindringtiefe der Strahlung relativ gering sein kann, ist eine Einrichtung vorgesehen, mit der ein Medium in den Behälter eingeströmt wird, um im inneren des Behälters einen Wirbel zu erzeugen. Das den Wirbel verursachende Medium ist dabei transparent für die elektro-magnetische Strahlung, so dass im Inneren des Wirbels eine berührungslose Messung zur Bestimmung einer Eigenschaft des Inhalts des Behälters durchgeführt werden kann.

Aus der WO 89/09391 ist ein Verfahren zur Detektion von Verunreinigungen in Kunststoff-Getränkebehältern bekannt. Der Boden der Behälter ist dabei an der Innenseite mit einem Sensor versehen, der eine fluoreszierende Substanz enthält, die durch Bestrahlung mit UV-Licht detektiert werden kann. Kommt der Sensor mit Kontaminationen in Kontakt, so trübt sich der Sensor ein und die UV-Strahlung gelangt nicht mehr auf die fluoreszierende Substanz des Sensors.

Die GB 1,182,413 betrifft ein Verfahren zum Untersuchen von Behältern auf Verunreinigungen. Ein Lichtstrahl wird dabei mittels einer Optik über die Innenfläche des Behälters geführt, wobei dann Zeile für Zeile die gesamte Innenwand des Behälters rasterartig beleuchtet wird. Die Strahlenquelle der D2 ist dabei eine kontinuierliche Strahlenquelle.

Die DE 42 23 269 betrifft eine Vorrichtung zur Identifikation von Verunreinigungen von Kunststoffflaschen, wobei ein UV-Strahlungsimpuls von außen auf den Behälterboden geleitet wird und anschließend das ebenfalls nach außen abgestrahlte Fluoreszenz-Spektrum analysiert wird. Aus dem Fluoreszenz-Spektrum werden dann z.B. Aussagen über in der Flasche befindliche Restflüssigkeiten getroffen.

Aus der EP 1 614 630 ist eine Vorrichtung zur Sterilisation von Behältern bekannt, bei der eine UV-Strahlungsquelle außerhalb der Behälter angeordnet ist und die Strahlung mittels eines Lichtwellenleiters über die Behälteröffnung in das Innere der Behälter geleitet wird. Am Ende des Lichtwellenleiters befindet sich ein Abstrahlelement, das eine räumliche Abstrahlung des UV-Lichts im Inneren des Behälters ermöglicht.

Aus der JP S52-135788 ist eine Vorrichtung zur Identifikation von Verunreinigungen in Gegenständen und Behältern bekannt. Dabei wird als Marker eine lumineszente Flüssigkeit verwendet, die sich an Verunreinigungen festsetzt und auf diese Weise eine nachfolgende Detektion der Verunreinigung ermöglicht. Zur Inspektion werden die Gengenstände dann in eine Dunkelkammer gefördert und dort auf ein Podest gesetzt. Das Podest wird zur Inspektion in eine Inspektionsposition angehoben.

WO8800862 A1 offenbart Methoden zur Unterscheidung zwischen kontaminierten und nicht kontaminierten Behältern.

Aufgabe der vorliegenden Erfindung ist es, die Zuverlässigkeit einer Inspektionseinrichtung für Behälter zu erhöhen ohne dabei die Verweildauer des Behälters in der Inspektionseinrichtung wesentlich zu erhöhen.

Diese Aufgabe wird erfindungsgemäß mit einer Vorrichtung gemäß Anspruch 1 gelöst. Dabei werden die Innenwände eines leeren Behälters mittels einer Strahlenquelle beleuchtet und die durch an den Innenwänden des Behälters anhaftende Verunreinigungen hervorgerufene Lumineszenz-Strahlung wird in einer geeigneten Einrichtung detektiert und anschließend ausgewertet. Da das üblicherweise für Behälter verwendete Material, eine geringe Transparenz für die anregende Strahlung hat, erfolgt die Beleuchtung der Innenwände über eine Öffnung, z.B. die Mündungsöffnung, des leeren Behälters.

Die Strahlenquelle ist vorzugsweise eine elektro-magnetische Strahlenquelle, z.B. eine Strahlungsquelle für Licht im sichtbaren Bereich, eine UV-A-, UV-B-, UV-C- oder Röntgen-Strahlungsquelle oder eine Kombination hiervon.

Es ist bekannt, dass UV-C-Strahlung eine keimtötende Wirkung hat. Durch den Einsatz von UV-C-Strahlung kann daher zusätzlich in vorteilhafter Weise ausgenutzt werden, dass leichte, organische Kontaminationen durch Bakterien, Sporen oder Pilze nicht nur detektiert, sondern gleichzeitig abgetötet werden können, so dass Behälter mit solchen Verunreinigungen nicht aus der Abfüll-Einrichtung ausgesondert werden müssen.

Die Strahlenquelle kann gepulst betrieben und so gesteuert werden, dass die Lichtimpulse nur dann abgegeben werden, wenn sich ein Behälter vor der Strahlungsquelle befindet. Durch die üblicherweise geringe Transparenz des Behältermaterials für die anregende Strahlung, dient der Behälter selbst als Strahlungsschirm, so dass wenn überhaupt nur wenig Strahlung nach außen gelangt. Eine zusätzliche Abschirmung der Inspektionsvorrichtung kann dann sehr einfach gestaltet oder sogar ganz weggelassen werden. Der Pulsbetrieb hat den weiteren Vorteil, dass dadurch die Bewegungsunschärfe der Detektionseinrichtungen reduziert wird.

Die Strahlungsquelle kann auch eine kontinuierliche Strahlenquelle sein die im Dauerbetrieb eingesetzt wir. Insbesondere geeignet sind zum Beispiel Leuchtstoffröhren oder Leuchtstofflampen.

Die Behälterwand von typischerweise in der Getränkeindustrie verwendeten Behältern ist nicht transparent für die anregende Strahlung, so dass die anregende Strahlung oder die Strahlenquelle selbst durch die Behälteröffnung ins Behälterinnere geleitet bzw. eingeführt werden muss, um die Behälterinnenwand zu bestrahlen.

Die Strahlenquelle ist vorzugsweise außerhalb des Behälters angeordnet und die anregende Strahlung wird z.B. über einen oder mehrere Spiegel durch die Mündungsöffnung in das Innere des Behälters geführt.

In einer weiteren nicht erfindungsgemäßen Ausführungsform kann eine Einrichtung vorgesehen sein, mit der ein Apparatekopf durch die Mündungsöffnung in den Behälter eingeführt wird. An dem Apparatekopf kann dabei zum Beispiel ein Lichtwellenleiter angebracht sein, über den die anregende Strahlung in den Behälter geführt wird. Alternativ, kann der Apparatekopf auch die Strahlungsquelle selbst umfassen. Der Apparatekopf kann auch Detektionseinrichtungen zur Detektion der reflektierten Strahlung umfassen.

Die von den Verunreinigungen ausgesandte Lumineszenz-Strahlung kann durch die Mündungsöffnung des Behälters aus dem Behälter herausgeführt werden und zum Beispiel mittels eines Spiegels auf eine Detektionseinrichtung gelenkt werden. Eine solche Anordnung hat den Vorteil, dass sie besonders einfach ausgestaltet ist, da nur eine einzige Einrichtung zur Detektion der Lumineszenz-Strahlung benötigt wird.

Vorteilhafterweise ist der Spiegel ein dichroitischer Spiegel, der die in den Behälter zu leitende einfallende Strahlung passieren lässt und die aus der Mündungsöffnung des Behälters austretende Lumineszenz-Strahlung, die eine höhere Wellenlänge als die anregende Strahlung aufweist, reflektiert und auf die Detektionsvorrichtung lenkt.

Der Teil der anregenden Strahlung, der aus dem Behälter wieder herausreflektiert wird, wird dagegen von dem dichroitischen Spiegel nicht abgelenkt und trifft damit nicht auf die Detektionseinrichtung. Optional kann zusätzlich ein Strahlungsfilter vor der Detektionseinrichtung vorgesehen sein, um UV-Strahlung oder um ganz allgemein ungewünschte Strahlungsfrequenzen selektiv zu blocken.

In einer bevorzugten Ausführungsform wird die von den Verunreinigungen ausgesandte Lumineszenz-Strahlung durch die Wände des Behälters aus dem Behälter herausgeführt und von einer oder mehreren Detektionsvorrichtungen, die um den Behälter angeordnet sind, aufgefangen. Diese Ausführungsform bietet sich an, wenn aufgrund der Geometrie des Behälters eine direkte Ausleuchtung der Behälterinnenwand über die Mündungsöffnung nicht erreicht werden kann. Voraussetzung hierfür ist natürlich, dass die Behälterwand zumindest für einen Teil der zu erwartende Lumineszenz-Strahlung transparent ist. Konventionelles Behälterglas erfüllt diese Voraussetzung.

Sowohl die Anordnung als auch die Anzahl der eingesetzten Detektionseinrichtungen ist bei dieser Ausführungsform frei wählbar. Entscheidend ist hierbei lediglich, dass ein Abbild der gesamten Behälterinnenwand erreicht wird, um eine Inspektion des gesamten Behälters auf Verunreinigungen zu gewährleisten.

Ein Herausführen der Lumineszenz-Strahlung aus dem Behälterinneren über die Behälterwand bietet sich auch bei Ausführungsformen an, bei denen die Strahlenquelle über die Behältermündung in den Behälter eingeführt wird und dadurch der Strahlengang der Lumineszenz-Strahlung durch die Behälteröffnung blockiert ist.

Die Einrichtungen zur Detektion der Lumineszenz-Strahlung sind vorzugsweise CCD-Kameras. Um Bewegungsunschärfe zu vermeiden bzw. zu reduzieren, können Shutterkameras mit kurzen Verschlusszeiten eingesetzt werden. Dies ist besonders von Vorteil, wenn die Strahlungsquelle im Dauerbetrieb arbeitet. Zur Erhöhung der Strahlungsintensität kann die anregende Strahlung mittels einer Linse auf die Mündungsöffnung gebündelt werden.

Die Erfindung betrifft außerdem ein Verfahren gemäß Anspruch 6 zur Inspektion von leeren Behältern auf Verunreinigungen. Das Verfahren umfasst die Schritte des Bestrahlens der Innenwände des Behälters mit einer Strahlungsquelle, des Detektierens der von eventuell vorhandenen Verunreinigungen abgestrahlten Lumineszenz-Strahlung mittels einer Detektionseinrichtung, und des Auswertens der detektierten Lumineszenz-Strahlung in einer Auswerteeinrichtung.

Wenn bei dem erfindungsgemäßen Verfahren eine starke UV-Strahlungsquelle, z.B. eine UV-C-Strahlungsquelle verwendet wird, ergibt sich der Vorteil, dass die Strahlung nicht nur zur Detektion der Verunreinigungen verwendet werden kann, sondern dass durch die Strahlung auch gleichzeitig Verunreinigungen, wie Keime, Bakterien, Pilze oder Sporen abgetötet werden.

Bei der Erfassung der Kontaminationen wird zudem eine hochgradige Empfindlichkeit erzeugt, die es z.B. erlaubt zwischen schwachen, aber großflächigen Kontaminationen, wie Verkeimung, und gröberen Verunreinigungen zu unterscheiden. Dünne Keimschichten können durch die Bestrahlung mit UV-C-Strahlung abgetötet werden, so dass es dann nicht nötig ist, einen solchen Behälter aus dem Abfüllprozess auszusondern. Werden bei einem Behälter dagegen gröbere Verunreinigungen erkannt, muss dieser Behälter erneut einer Reinigung unterzogen werden.

Gemäß einer weiteren bevorzugten Ausführungsform, werden kontaminierte Behälter nur dann ausgesondert, wenn die detektierte Lumineszenz-Strahlung einen vorher festgelegten Schwellenwert übersteigt. Zur Erkennung und Charakterisierung der Kontaminationen wird das auf der Lumineszenz-Strahlung basierende Bild des Behälters einer Bildauswerte-Elektronik zugeführt, die zum Beispiel besonders helle Bereiche oder farbliche Unterschiede erkennt. Die Auswertung kann zum Beispiel durch vergleich mit abgespeicherten Daten oder Mustern erfolgen. Bei Abweichungen oder Schwellwert-Überschreitungen gibt die Auswerteelektronik ein Fehlersignal zurück, welches dann gegebenenfalls zur Aussonderung des betreffenden Behälters führt.

Die vorliegende Erfindung ist dazu geeignet, sowohl fluoreszierende als auch phosphoreszierende Verunreinigungen zu detektieren.

Die vorliegende Erfindung kann außerdem zur Inspektion von Behältern aus beliebigem Material eingesetzt werden. Besonders vorteilhaft lässt sich die Erfindung bei Behältern aus Materialien einsetzen, die intransparent für die anregende Strahlung, aber transparent für die Lumineszenz-Strahlung sind. Besonders geeignet ist die Erfindung daher zur Anwendung bei Behältern aus Glas oder transparenten Kunststoffen wie z.B. PET.

Anhand nachfolgender Abbildungen werden das Verfahren und die Vorrichtung der vorliegenden Erfindung näher erläutert. Es zeigen:
- Fig. 1: ein Prinzipschema einer ersten Vorrichtung zur Inspektion leerer Behälter;
- Fig. 2: ein Prinzipschema einer zweiten Vorrichtung zur Inspektion leerer Behälter;
- Fig. 3: Draufsicht der Vorrichtung aus Fig. 2

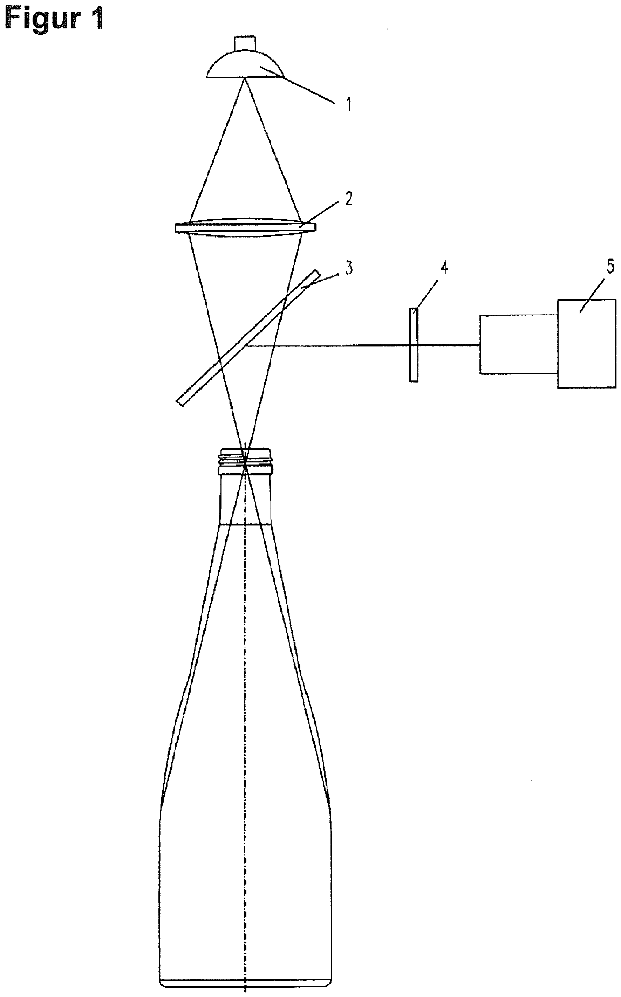

Die in Fig. 1 abgebildete Vorrichtung umfasst eine elektro-magnetische Strahlungsquelle 1, deren Strahlung mittels einer Linse 2 im Bereich der Mündung einer Flasche gebündelt wird. Die Strahlung durchläuft dabei einen zwischen der Linse 2 und der Flaschenmündung angeordneten dichroitischen Spiegel 3. Der dichroitische Spiegel 3 ist so ausgebildet, dass er die anregende Strahlung passieren lässt, aber die zu erwartende langwelligere Lumineszenz-Strahlung reflektiert.

Wenn die Flasche keine Verunreinigungen aufweist, wird ein Teil der anregenden Strahlung wieder aus der Flaschenöffnung zurückreflektiert und passiert den dichroitischen Spiegel 3 ohne auf die CCD-Kamera 5 abgelenkt zu werden.

Befindet sich in der Flasche jedoch eine lumineszente Verunreinigung, also eine Verunreinigung, die auf die anregende Strahlung mit einer Lumineszenz-Erscheinung reagiert, so verlässt ein Teil der von der Verunreinigung abgegebenen Lumineszenz-Strahlung die Flasche durch die Mündung und trifft auf den dichroitischen Spiegel 3. Der dichroitischen Spiegel 3 reflektiert diese langwelligere Lumineszenz-Strahlung auf die CCD-Kamera 5, in der die Strahlung dann detektiert wird.

Zusätzlich kann ein Filter 4 vorgesehen sein, um zu verhindern, dass Teile der anregenden Strahlung auf die CCD-Kamera gelangen, oder um nur bestimmte Frequenzbereiche selektiv passieren zu lassen.

Die Ausführungsform, die in Fig. 1 dargestellt ist, eignet sich besonders zur Inspektion von Behältern, bei denen über die Behälteröffnung der gesamte Innenraum des Behälters ausgeleuchtet werden kann, also z.B. Flaschen mit langen, sich langsam weitenden Flaschenhälsen oder Behälter mit großen Mündungsöffnungen.

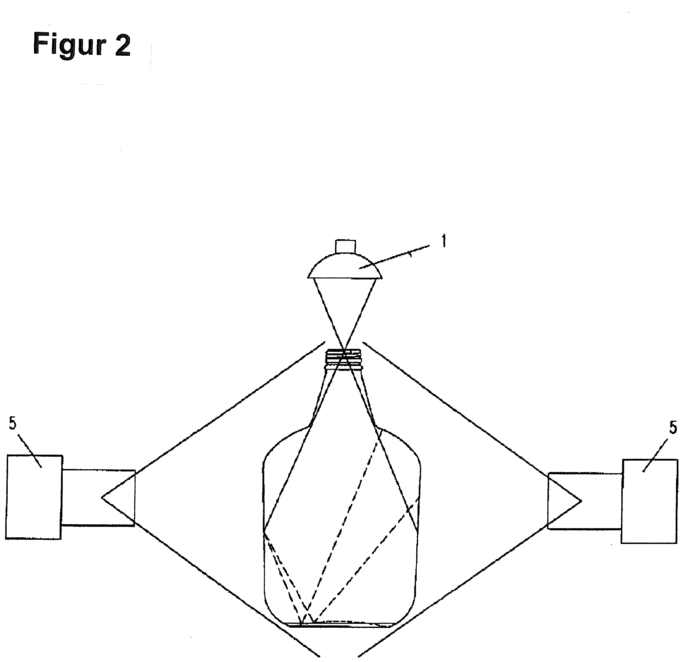

Bei Behältern, die aufgrund ihrer Geometrie ein Ausleuchten der Innenwände über die Mündungsöffnung nicht zulassen, bietet sich ein modifizierter Aufbau an, wie er in Fig. 2 gezeigt ist. Vorrausetzung hierbei ist jedoch, dass die Behälterwand aus einem für die Lumineszenz-Strahlung transparenten Material besteht.

In der Vorrichtung gemäß Fig. 2 wird die Strahlungsquelle ebenfalls über dem zu untersuchenden Behälter positioniert. Die anregende Strahlung wird über die Mündungsöffnung des Behälters in das Behälterinnere geleitet. Wie in Fig. 2 angedeutet wird die einfallende Strahlung an den inneren Seitenwänden und dem Boden des zu untersuchenden Behälters reflektiert, so dass auch bei dieser Vorrichtung der gesamte Innenraum des Behälters ausgeleuchtet wird.

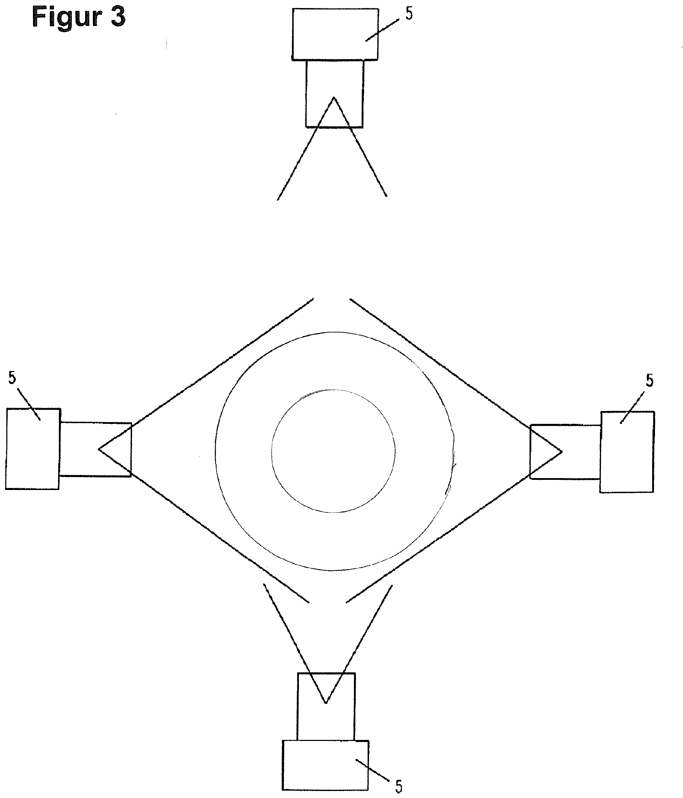

Befindet sich bei der Vorrichtung der Fig.2 in dem Behälter eine lumineszente Verunreinigung, so gibt diese Verunreinigung aufgrund der anregenden Strahlung wiederum Lumineszenz-Strahlung ab. Wie oben diskutiert, ist die Wellenlänge der Lumineszenz-Strahlung größer als die der anregenden Strahlung und kann daher, wenn der Behälter aus einem Material besteht, der für den Frequenzbereich der Lumineszenz-Strahlung transparent ist, auch direkt durch die Behälterwand aus dem Behälter austreten. Zur Detektion dieser Lumineszenz-Strahlung sind außerhalb des Behälters angebrachte Detektionseinrichtungen wie z.B. CCD - Kameras vorgesehen. Die Anordnung der Detektionseinrichtungen ist in der Draufsicht der Fig. 3 dargestellt. Bei dieser Anordnung sind jeweils 2 CCD-Kameras paarweise gegenüber angeordnet. Die Anzahl und Anordnung der Detektionseinrichtungen ist jedoch frei wählbar, solange gewährleistet ist, dass ein vollständiges Bild des Behälters erzielt wird. Es ist auch möglich, Detektionseinrichtungen unterhalb oder oberhalb der zu untersuchenden Behälter anzubringen.

Ein zusätzlicher Filter zum Blocken der anregenden Strahlung ist bei dieser Ausführungsform nicht nötig, wenn das Material des Behälters intransparent für die anregende Strahlung ist. Die Ausführungsform aus Fig. 2 eignet sich z.B. besonders für die Untersuchung von Glasbehältern mittels UV-Strahlung. Das Behälterglas ist typischerweise nahezu undurchlässig für UV-Strahlung. Die von Verunreinigungen abgestrahlte Lumineszenz-Strahlung hat dagegen eine größere Wellenlänge, die überwiegend im sichtbaren Bereich liegt und kann daher die Behälterwand problemlos passieren.

## Patentansprüche

1. Vorrichtung zur Inspektion von leeren Behältern auf Verunreinigungen zum Einsatz in automatischen Abfüllanlagen, in denen die Behälter mit hohen Geschwindigkeiten von bis zu 90.000 Flaschen pro Stunde transportiert werden, umfassend
eine Strahlungsquelle (1) zur Generation einer anregenden Strahlung, wobei die anregende Strahlung auf die Innenwand eines Behälters geleitet wird und zu detektierende Verunreinigungen zur Abgabe von Lumineszenz-Strahlung anregt,
mindestens eine Einrichtung (5) zur Detektion der von den Verunreinigungen abgegebenen Lumineszenz-Strahlung, sowie
eine Einrichtung zur Auswertung der detektierten Lumineszenz-Strahlung,
wobei die anregende Strahlung durch die Behälteröffnung in das Innere des Behälters geleitet wird, wobei
die Strahlungsquelle so ausgeführt ist, dass der gesamte Innenraum des Behälters ausgeleuchtet wird, und wobei
die Strahlungsquelle (1) eine im Pulsbetrieb arbeitende Strahlungsquelle ist, oder
die Detektionseinrichtung (5) eine Shutterkamera ist.

2. Vorrichtung gemäß Anspruch 1, wobei die Strahlungsquelle (1), eine elektro-magnetische Strahlungsquelle, vorzugsweise eine Strahlungsquelle für Licht im sichtbaren Bereich, eine UV-A-, UV-B-, UV-C- oder Röntgen-Strahlungsquelle oder eine Kombination hiervon, ist.

3. Vorrichtung gemäß einem der vorangehenden Ansprüche, wobei die von den Verunreinigungen ausgesandte Lumineszenz-Strahlung durch die Behälteröffnung aus dem Behälter herausgeführt und auf die Detektionsvorrichtung (5) gelenkt wird.

4. Vorrichtung gemäß Anspruch 3, umfassend einen dichroitischen Spiegel (3) der die anregende Strahlung passieren lässt und die aus der Behälteröffnung austretende Lumineszenz-Strahlung auf die Detektionsvorrichtung (5) lenkt.

5. Vorrichtung gemäß einem der Ansprüche 1 bis 2, wobei die von den Verunreinigungen ausgesandte Lumineszenz-Strahlung durch die Wände des Behälters aus dem Behälter herausgeführt und von einer oder mehreren Detektionsvorrichtungen (5), die um den Behälter angeordnet sind aufgefangen wird.

6. Verfahren zur Inspektion von leeren Behältern auf Verunreinigungen in automatischen Abfüllanlagen, in denen die Behälter mit hohen Geschwindigkeiten von bis zu 90.000 Flaschen pro Stunde transportiert werden
, umfassend die Schritte:
Bestrahlen der Innenwände des Behälters mit einer anregenden Strahlung, wobei die anregende Strahlung zu detektierende Verunreinigungen zur Abgabe von Lumineszenz-Strahlung anregt, und wobei die anregende Strahlung durch die Behälteröffnung in das Innere des Behälters geleitet wird
Detektieren der von den Verunreinigungen abgestrahlten Lumineszenz-Strahlung mittels einer Detektionseinrichtung (5), und
Auswerten der detektierten Lumineszenz-Strahlung in einer Auswerteeinrichtung, wobei
mit der Strahlungsquelle (1) der gesamte Innenraum des Behälters ausgeleuchtet wird, und wobei
entweder die Strahlungsquelle (1) im Pulsbetrieb betrieben wird, oder
die Detektionseinrichtung (5) eine Shutterkamera ist.

7. Verfahren gemäß Anspruch 6, wobei die Strahlungsquelle (1), eine elektro-magnetische Strahlungsquelle, vorzugsweise eine Strahlungsquelle für Licht im sichtbaren Bereich, eine UV-A-, UV-B-, UV-C- oder Röntgen-Strahlungsquelle oder eine Kombination hiervon, ist.

8. Verfahren gemäß einem der Ansprüche 6 bis 7, wobei die verwendete Strahlungsquelle mindestens einen Anteil UV-C-Strahlung aufweist, so dass organische Verunreinigungen wie Pilze, Sporen und Bakterien detektiert und gleichzeitig unschädlich gemacht werden.

9. Verfahren gemäß einem der Ansprüche 6 bis 8, wobei ein kontaminierter Behälter nur dann ausgesondert wird, wenn die detektierte Lumineszenz-Strahlung einen vorher festgelegten Schwellenwert übersteigt.

10. Verfahren gemäß einem der Ansprüche 6 bis 9, wobei sowohl die Fluoreszenz als auch Phosphoreszenz der Verunreinigungen ausgewertet wird.

## Claims

1. Apparatus for inspecting empty containers for contaminants for use in automatic filling systems in which the containers are transported at high speeds of up to 90,000 bottles per hour, comprising
a radiation source (1) for generating an exciting radiation, wherein the exciting radiation is directed on to the inner wall of a container and excites contaminants to be detected in such a way that they emit luminescent radiation,
at least one device (5) for detecting the luminescent radiation emitted by the contaminants, and
a device for analysing the detected luminescent radiation,
wherein the exciting radiation is directed through the container opening into the interior of the container, wherein
the radiation source is configured to illuminate the entire internal space of the container, and wherein
the radiation source (1) is operated in pulsed mode, or the detection device (5) is a shutter camera.

2. Apparatus according to claim 1, wherein the radiation source (1) is an electromagnetic radiation source, preferably a radiation source for light in the visible range, a UV-A, UV-B, UV-C or X-ray radiation source or a combination thereof.

3. Apparatus according to one of the preceding claims, wherein the luminescent radiation emitted by the contaminants is guided out of the container through the container opening and directed on to the detection device (5).

4. Apparatus according to claim 3, comprising a dichroic mirror (3) which lets through the exciting radiation and directs the luminescent radiation exiting from the container opening on to the detection device (5).

5. Apparatus according to one of claims 1 to 2, wherein the luminescent radiation emitted by the contaminants is guided out of the container through the walls of the container and collected by one or more detection devices (5), which are arranged around the container.

6. Method for inspecting empty containers for contaminants for use in automatic filling systems in which the containers are transported at high speeds of up to 90,000 bottles per hour, comprising the steps:
irradiating the inner walls of the container with an exciting radiation, wherein the exciting radiation excites contaminants to be detected in such a way that they emit luminescent radiation and wherein the exciting radiation is directed through the container opening into the interior of the container,
detecting the luminescent radiation emitted by the contaminants using a detection device (5), and
analysing the detected luminescent radiation in an analysing device,
wherein the radiation source (1) illuminates the entire internal space of the container, and
wherein either the radiation source (1) is operated in pulsed mode, or the detection device (5) is a shutter camera.

7. Method according to claim 6, wherein the radiation source (1) is an electromagnetic radiation source, preferably a radiation source for light in the visible range, a UV-A, UV-B, UV-C or X-ray radiation source or a combination thereof.

8. Method according to one of claims 6 to 7, wherein the radiation source used has at least a proportion of UV-C radiation, so that organic contaminants such as fungi, spores and bacteria are detected and at the same time rendered harmless.

9. Method according to one of claims 6 to 8, wherein a contaminated container is rejected only if the detected luminescent radiation exceeds a previously set limit value.

10. Method according to one of claims 6 to 9, wherein both the fluorescence and the phosphorescence of the contaminants are analysed.

## Revendications

1. Dispositif d'inspection de contenants vides à la recherche d'impuretés, destiné à être utilisé dans des installations de remplissage automatiques dans lesquelles les contenants sont transportés à des vitesses élevées allant jusqu'à 90 000 bouteilles par heure, comprenant
une source de rayonnement (1) destinée à générer un rayonnement d'excitation, le rayonnement d'excitation étant dirigé sur la paroi intérieure d'un contenant et excitant des impuretés à détecter afin qu'elles émettent un rayonnement luminescent,
au moins un appareil (5) destiné à détecter le rayonnement luminescent émis par les impuretés, ainsi que
un appareil destiné à évaluer le rayonnement luminescent détecté,
dans lequel le rayonnement d'excitation est dirigé vers l'intérieur du contenant par l'ouverture du contenant, dans lequel
la source de rayonnement est réalisée de telle manière que la totalité de l'espace intérieur du contenant est illuminée, et dans lequel
la source de rayonnement (1) est une source de rayonnement fonctionnant en mode pulsé, ou
l'appareil de détection (5) est un appareil de prise de vues à obturateur.

2. Dispositif selon la revendication 1, dans lequel la source de rayonnement (1) est une source de rayonnement électromagnétique, de préférence une source de rayonnement pour la lumière dans le domaine visible, une source de rayonnement UV-A, UV-B, UV-C ou X ou une combinaison de celles-ci.

3. Dispositif selon l'une des revendications précédentes, dans lequel le rayonnement luminescent émis par les impuretés est guidé hors du contenant par l'ouverture du contenant et est dirigé sur le dispositif de détection (5).

4. Dispositif selon la revendication 3, comprenant un miroir dichroïque (3) qui laisse passer le rayonnement d'excitation et dirige le rayonnement luminescent sortant de l'ouverture du contenant sur le dispositif de détection (5).

5. Dispositif selon l'une des revendications 1 à 2, dans lequel le rayonnement luminescent émis par les impuretés est guidé hors du contenant par les parois du contenant et est capté par un ou plusieurs dispositifs de détection (5) qui sont disposés autour du contenant.

6. Procédé d'inspection de contenants vides à la recherche d'impuretés dans des installations de remplissage automatiques dans lesquelles les contenants sont transportés à des vitesses élevées allant jusqu'à 90 000 bouteilles par heure,
comprenant les étapes consistant à :
exposer les parois intérieures du contenant à un rayonnement d'excitation, le rayonnement d'excitation excitant des impuretés à détecter afin qu'elles émettent un rayonnement luminescent, et le rayonnement d'excitation étant dirigé vers l'intérieur du contenant par l'ouverture du contenant
détecter le rayonnement luminescent émis par les impuretés au moyen d'un appareil de détection (5), et
évaluer le rayonnement luminescent détecté dans un appareil d'évaluation, dans lequel
la totalité de l'espace intérieur du contenant est illuminée avec la source de rayonnement (1), et dans lequel
soit la source de rayonnement (1) fonctionne en mode pulsé, soit
l'appareil de détection (5) est un appareil de prise de vues à obturateur.

7. Procédé selon la revendication 6, dans lequel la source de rayonnement (1) est une source de rayonnement électromagnétique, de préférence une source de rayonnement pour la lumière dans le domaine visible, une source de rayonnement UV-A, UV-B, UV-C ou X ou une combinaison de celles-ci.

8. Procédé selon l'une des revendications 6 à 7, dans lequel la source de rayonnement utilisée présente au moins une part de rayonnement UV-C, de telle sorte que des impuretés organiques telles que des champignons, des spores et des bactéries sont détectées et simultanément rendues inoffensives.

9. Procédé selon l'une des revendications 6 à 8, dans lequel un contenant contaminé n'est éliminé que quand le rayonnement luminescent détecté dépasse une valeur seuil préalablement définie.

10. Procédé selon l'une des revendications 6 à 9, dans lequel aussi bien la fluorescence que la phosphorescence des impuretés sont évaluées.
